# EUROPEAN PATENT SPECIFICATION

(11) **EP 3 351 586 B1**
(45) Date of publication and mention of the grant of the patent: **06.05.2020**
(21) Application number: 16846374.3
(22) Date of filing: 08.09.2016
(51) Int. Cl.: C08J 5/00, B29C 43/34, C07K 14/435, C08H 1/00, C12N 15/09, B29C 43/00, B29C 43/52

(54) **MOLDED ARTICLE AND METHOD FOR PRODUCING MOLDED ARTICLE**
FORMARTIKEL UND VERFAHREN ZUR HERSTELLUNG EINES FORMARTIKELS
ARTICLE MOULÉ ET SON PROCÉDÉ DE FABRICATION

(30) Priority: 18.09.2015 JP 2015185766
(43) Date of publication of application: 25.07.2018
(73) Proprietor: Spiber Inc., Yamagata 997-0052 (JP); Kojima Industries Corporation, Toyota-shi, Aichi 471-8588 (JP)
(72) Inventor: SUGAHARA Junichi, Tsuruoka-shi Yamagata 997-0052 (JP); SEKIYAMA Kaori, Tsuruoka-shi Yamagata 997-0052 (JP); ABE Ayumi, Tsuruoka-shi Yamagata 997-0052 (JP); SHIMOKATA Junichi, Tsuruoka-shi Yamagata 997-0052 (JP); NOBA Junichi, Toyota-shi Aichi 470-0441 (JP); HIRAI Shinji, Muroran-shi Hokkaido 050-8585 (JP)
(74) Representative: Hoffmann Eitle
(86) International application number: PCT/JP2016/076500
(87) International publication number: WO 2017/047503

(56) References cited:
- WO-A1-2008/071226
- WO-A1-2012/165476
- WO-A1-2014/062134
- WO-A1-2014/103799
- JP-A- 2001 315 056
- JP-A- 2002 001 648
- US-B2- 8 666 471

## Description

### Technical Field

The present invention relates to a molded article and a method for producing a molded article.

### Background Art

Inorganic glass is excellent in terms of transparency or thermal resistance and is thus used as window members in a number of uses including car uses. On the other hand, inorganic glass is poor in terms of formability to three-dimensional curves and has a great specific weight, and thus synthetic resin windows made of an acrylic resin or a polycarbonate resin which does not easily cause the above-described problems have been proposed (for example, refer to Patent Literature 1). In addition, due to a rising demand for environmental protection, bioplastics which are petroleum-free materials have been thus far drawing attention, as a transparent and high-strength material, "TERRAMAC" (registered trademark) is known, and transparent materials having 99.5% of a polylactic acid percentage and 4.3 GPa of a bending elastic modulus have been realized.

### Citation List

### Patent Literature

[Patent Literature 1] JP H6-156071 A Patent document US 8,666,471 B2 discloses a biomedical device having dissolvable films of silk fibroin and transparency.

### Summary of Invention

### Technical Problem

Regarding car window glass, the weight has an influence on gas mileage and, furthermore, may cause a problem of environmental burden, and thus weight reduction is extremely important. Therefore, an object of the present invention is to provide a highly transparent formed article which can weigh less than conventional synthetic resins and a production method therefor.

### Solution to Problem

The present invention provides a molded article of a composition comprising a polypeptide wherein the molded article has more than 4.3 GPa of a bending elastic modulus and transparency.

The molded article has transparency and thus can be applied to window uses with no problems. In addition, the molded article exhibits a bending elastic modulus as high as more than 4.3 GPa, and thus, in the case of being formed to a panel shape, the molded article is capable of producing a strength that is equal to or higher than those of conventional synthetic resins with a thinner thickness, which significantly contributes to weight reduction. The specific weight of the molded article is also equal to those of synthetic resins such as acrylic resins or polycarbonate resins and is overwhelmingly lighter than that of inorganic glass. Therefore, in a number of uses including car uses, it is possible to reduce environmental burden by weight reduction. Meanwhile, the bending elastic modulus is preferably 4.7 GPa or more, more preferably 5.0 GPa or more, and still more preferably 5.2 GPa or more. The upper limit of the bending elastic modulus is not limited, but can be set to, for example, 15.0 GPa or less. The bending elastic modulus can be set to 10.0 GPa or less and, furthermore, 7.0 GPa or less.

As the polypeptide, the composition preferably contains at least one kind selected from the group consisting of natural spider silk protein and polypeptides derived from natural spider silk protein. The natural spider silk protein or the polypeptide derived from natural spider silk protein is significantly excellent in terms of both the bending elastic modulus and the bending strength and, when added as the polypeptide, enables additional weight reduction and additional function improvement.

As the polypeptide, the composition may further contain silk fibroin, soybean protein, casein, keratin, collagen, and milk whey protein. When these are jointly used with the natural spider silk protein or the polypeptide derived from natural spider silk protein, it becomes easy to design the performance of the molded article depending on intended uses.

The molded article may be provided as a hot press-molded article. The molded article refers to an article or the like formed using a casting mold (mold), but articles having a superior bending elastic modulus can be produced by hot pressing procedure.

The molded article can be produced using a production method comprising a step of hot pressing the above-described composition comprising the polypeptide.

### Advantageous Effects of Invention

According to the present invention, a highly transparent formed article which can weigh less than synthetic resins of the related art and a production method therefor are provided.

### Brief Description of Drawings

Fig. 1 is a schematic cross-sectional view of a press molding machine.
Fig. 2 illustrates schematic cross-sectional views of the press molding machine (a) before the introduction of a composition, (b) immediately after the introduction of the composition, and (c) with the composition in a state of being hot pressed.
Fig. 3 is a photograph of a molded article of Example 1.

### Description of Embodiments

Hereinafter, a preferred embodiment of the present invention will be described. However, the present invention is not limited to the following embodiment by any means.

A molded article according to the embodiment is made of a composition comprising a polypeptide and the polypeptide preferably includes natural spider silk protein and/or a polypeptide derived from natural spider silk protein (hereinafter, in some cases, these will be collectively referred to as "spider silk polypeptide"). The molded article may contain, as the polypeptide, a polypeptide other than the spider silk polypeptide, and examples of the above-described polypeptide include silk fibroin, soybean protein, casein, keratin, collagen, and milk whey protein.

The molded article can be obtained by introducing the above-described composition into a casting mold (mold) and carrying out forming or the like, and, in the forming step, heating and/or pressing is possible. The composition has, typically, a shape of a powder form (lyophilized powder or the like) or a fibrous shape (a fiber or the like obtained by spinning), and the molded article may be a fused article of a composition comprising a polypeptide having the above-described shape.

Examples of the natural spider silk protein that is used as the polypeptide constituting the molded article include a major dragline silk protein and a flagelliform silk protein.

The major dragline silk protein is produced from spider's major ampullate glands and has a characteristic of excellent toughness. Examples of the major dragline silk protein include major ampullate spidroin MaSp1 or MaSp2 derived from Nephilaclavipes, ADF3 or ADF4 derived from Araneus diadematus, and the like.

The flagelliform silk protein is produced from spider's flagelliform glands, and examples of the flagelliform silk protein include flagelliform silk protein derived from Nephilaclavipes.

Examples of the polypeptides derived from natural spider silk protein include recombinant spider silk protein, for example, mutants, analogues, derivatives, and the like of natural spider silk protein. The above-described polypeptide is particularly preferably the recombinant spider silk protein of the major dragline silk protein.

As the polypeptide, in addition to the above-described spider silk polypeptide, silk fibroin, soybean protein, casein, keratin, collagen, or milk whey protein can be jointly used. The amount of silk protein or the like in the case of being jointly used needs to be an amount at which the transparency of the molded article can be ensured and is, for example, preferably 20 parts by mass or less, preferably 10 parts by mass or less, and more preferably 5 parts by mass or less with respect to 100 parts by mass of the spider silk polypeptide.

As the silk fibroin, it is possible to use sericin-removed silk fibroin and/or sericin-unremoved silk fibroin. Silk is a fiber that is obtained from cocoons made by silkworms that are larvae of Bombyx mori, and a cocoon filament is a single filament formed of two fibroin threads being covered with a gluey substance (sericin) on the outside. Fibroin is constituted of a number of fibrils, and the outside of fibroin is covered with four layers of sericin, thereby constituting one cocoon filament. Practically, sericin on the outside is dissolved and removed by means of refining, and cocoon filaments are used in clothing uses as silk filaments. The specific weight of silk is 1.33, the average fineness is 3.3 decitex, and the fiber length is, generally, approximately 1,300 to 1,500 m.

The silk fibroin is preferably silk fibroin lyophilized powder obtained by using cocoons of natural or domesticated silkworm or used or discarded silk cloth as a raw material and purifying silk fibroin from which sericin covering silk fibroin, other fat components, and the like are removed (lyophilized powder of sericin-removed silk fibroin). Meanwhile, as the silk fibroin, silk fibroin from which sericin and the like are not removed (sericin-unremoved silk fibroin) can also be used.

The molded article may be a molded article including the polypeptide alone and also may be a molded article in which additive components (for example, a plasticizer, a nucleating agent, an antioxidant, a coloring agent, a filler such as lamellar silicate or basic calcium phosphate, water, synthetic resins, and the like) are added to the polypeptide. In a case in which additive components of a plasticizer and the like are used, it is preferable to set the amount thereof to 50% by mass or less of the total amount of the polypeptides. In addition, foreign substances that are generated in a process of obtaining the polypeptide may also be included. Meanwhile, the molded article exhibits the effects of the present invention even in a case in which the above-described additive components are not added thereto.

The molded article has transparency. The transparency can be visually determined, but the molded article preferably has a transmittance of 50% or more in a case in which an optical transmittance measurement instrument is used and, for example, the cumulative time is set to 0.1 seconds in a wavelength range of 220 to 800 nm.

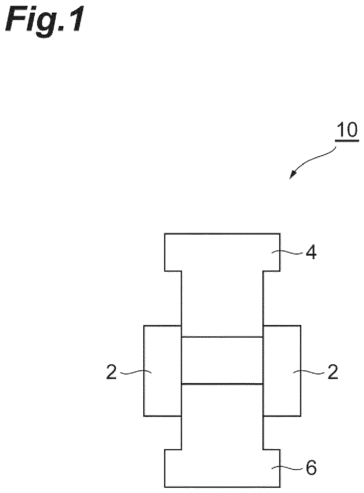

The molded article can be produced using a press molding machine. Fig. 1 is a schematic cross-sectional view of a press molding machine that can be used to produce the molded article. A press molding machine 10 illustrated in Fig. 1 includes a die 2 in which a through hole is formed and which can be heated and an upper side pin 4 and a lower side pin 6 which can be moved up and down in the through hole of the die 2. The molded article can be obtained by introducing the composition including the spider silk polypeptide into a cavity formed by inserting the upper side pin 4 or the lower side pin 6 into the die 2, and pressing the composition with the upper side pin 4 and the lower side pin 6 while heating the die 2.

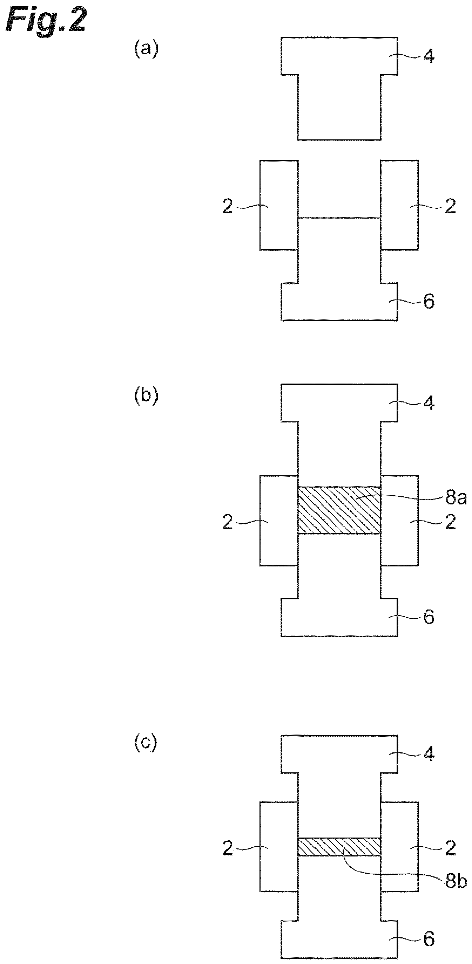

Fig. 2 illustrates step views of obtaining the molded article and are schematic cross-sectional views of the press molding machine (a) before the introduction of the composition, (b) immediately after the introduction of the composition, and (c) with the composition in a state of being heated and pressed. As illustrated in Fig. 2(a), the composition is introduced into the through hole in a state in which only the lower side pin 6 is inserted into the through hole of the die 2, and, as illustrated in Fig. 2(b), the upper side pin 4 is inserted into the through hole of the die 2 and moved down, and the heating of the die 2 is initiated, thereby heating and pressing a composition 8a which is not yet heated and pressed in the through hole. The upper side pin 4 is moved down until a predetermined pressurization force is reached, and heating and pressing are continued until the composition reaches a predetermined temperature in a state illustrated by Fig. 2(c), thereby obtaining a composition 8b which has been hot pressed. After that, the temperature of the die 2 is lowered using a cooler (for example, a spot cooler), the upper side pin 4 or the lower side pin 6 is removed from the die 2 when the composition 8b reaches a predetermined temperature, and the resulting content is taken out, thereby obtaining a molded article. The pressing may be carried out by moving down the upper side pin 4 in a state in which the lower side pin 6 is fixed, but it is also possible to move down the upper side pin 4 and move up the lower side pin 6 at the same time.

The heating is preferably carried out at 80°C to 300°C, more preferably carried out at 100°C to 180°C, and still more preferably carried out at 100°C to 130°C. The pressing is preferably carried out at 5 kN or more, more preferably carried out at 10 kN or more, and still more preferably carried out at 20 kN or more. In addition, the time during which the treatment is continued under predetermined hot pressing conditions after the conditions are satisfied (temperature retention condition) is preferably 0 to 100 minutes, more preferably 1 to 50 minutes, and still more preferably 5 to 30 minutes.

The molded article of the composition comprising the polypeptide is preferably produced using the polypeptide derived from natural spider silk protein, and thus the production method will be described below in detail.

Examples of the polypeptide derived from the major dragline silk protein which serves as a raw material of the molded article include polypeptides including two or more, preferably including five or more, and more preferably including 10 or more units of an amino-acid sequence represented by Formula 1: REP1-REP2 (1). Alternatively, the polypeptide derived from the major dragline silk protein may be a polypeptide which includes a unit of the amino-acid sequence represented by Formula 1: REP1-REP2 (1) and in which a C terminal sequence is an amino-acid sequence represented by any of SEQ ID Nos. 1 to 3 or an amino-acid sequence having 90% or more homology with the amino-acid sequence represented by any of SEQ ID Nos. 1 to 3. Meanwhile, in the polypeptide derived from the major dragline silk protein, the units of the amino-acid sequence represented by Formula 1: REP1-REP2 (1) may be identical to or different from each other. In a case in which the production of recombinant protein is carried out using a microbe such as Escherichia coli as a host, the molecular weight of the polypeptide derived from the major dragline silk protein is preferably 500 kDa or less, more preferably 300 kDa or less, and still more preferably 200 kDa or less from the viewpoint of productivity.

In Formula 1, REP1 represents polyalanine. In REP1, alanines that are continuously arranged are preferably two or more residues, more preferably three or more residues, still more preferably four or more residues, and particularly preferably five or more residues. In addition, in REP1, alanines that are continuously arranged are preferably 20 or less residues, more preferably 16 or less residues, still more preferably 12 or less residues, and particularly preferably 10 or less residues. In Formula 1, REP2 is an amino-acid sequence made of 10 to 200 residues of amino acid, and the total residue number of glycine, serine, glutamine, and alanine in the amino-acid sequence is 40% or more, preferably 60% or more, and more preferably 70% or more of the total number of amino acid residues.

In the major dragline silk protein, REP1 corresponds to a crystalline region that forms a crystalline β sheet in the fiber, and REP2 corresponds to an amorphous region which is more flexible and lacks a regular structure in a majority area in the fiber. In addition, [REP1-REP2] corresponds to a repetitive region made up of crystalline regions and amorphous regions (repetitive sequence) and is the characteristic sequence of dragline silk protein.

An amino-acid sequence represented by SEQ ID No. 1 is the same as an amino-acid sequence made of 50 residues of amino acid at the C terminal of an amino-acid sequence of ADF3 (NCBI accession number: AAC47010, GI: 1263287), and an amino-acid sequence represented by SEQ ID No. 2 is the same as an amino-acid sequence obtained by removing 20 residues from the C terminal of the amino-acid sequence represented by SEQ ID No. 1, and an amino-acid sequence represented by SEQ ID No. 3 is the same as an amino-acid sequence obtained by removing 29 residues from the C terminal of the amino-acid sequence represented by SEQ ID No. 1.

As the polypeptide including two or more units of the amino-acid sequence represented by Formula 1: REP1-REP2 (1), it is possible to use, for example, a polypeptide made of an amino-acid sequence represented by SEQ ID No. 7. The polypeptide made of the amino-acid sequence represented by SEQ ID No. 7 is an amino-acid sequence of ADF3 (NCBI accession number: AAC47010, GI: 1263287) having an amino-acid sequence (SEQ ID No. 4) made up of an initiation codon, a His10 tag, and a Human rhinovirus 3C protease (HRV3C protease) recognition site added to an N terminal which is mutated so that translation ends at the 543^{rd} amino acid residue.

In addition, as the polypeptide including two or more units of the amino-acid sequence represented by Formula 1: REP1-REP2 (1), it is possible to use a protein which is made of the amino-acid sequence represented by SEQ ID No. 7 in which one or a plurality of amino acids is substituted, deleted, inserted, and/or added, and has a repetitive region made up of crystalline regions and amorphous regions. In the present invention, "one or a plurality" means, for example, 1 to 40, 1 to 35, 1 to 30, 1 to 25, 1 to 20, 1 to 15, 1 to 10, or 1 or several. In addition, in the present invention, "one or several" means 1 to 9, 1 to 8, 1 to 7, 1 to 6, 1 to 5, 1 to 4, 1 to 3, 1 or 2, or 1.

In addition, examples of the polypeptide including two or more units of the ammo-acid sequence represented by Formula 1: REP1-REP2 (1) include ADF4-derived recombinant protein having an amino-acid sequence represented by SEQ ID No. 8. The amino-acid sequence represented by SEQ ID No. 8 is an amino-acid sequence obtained by adding the amino-acid sequence (SEQ ID No. 4) made up of an initiation codon, a His 10 tag, and a Human rhinovirus 3C protease (HRV3C protease) recognition site to an N terminal of a partial amino-acid sequence of ADF4 (NCBI accession number: AAC47011, GI: 1263289) procured from NCBI database. In addition, as the polypeptide including two or more units of the amino-acid sequence represented by Formula 1: REP1-REP2 (1), a polypeptide which is made of the amino-acid sequence represented by SEQ ID No. 8 in which one or a plurality of amino acids is substituted, deleted, inserted, and/or added, and has a repetitive region made up of crystalline regions and amorphous regions may be used. In addition, examples of the polypeptide including two or more units of the amino-acid sequence represented by Formula 1: REPI-REP2 (1) include MaSp2-derived recombinant protein having an amino-acid sequence represented by SEQ ID No. 9. The amino-acid sequence represented by SEQ ID No. 9 is an amino-acid sequence obtained by adding an amino-acid sequence made up of an initiation codon, a His 10 tag, and a Human rhinovirus 3C protease (HRV3C protease) recognition site to an N terminal of a partial amino-acid sequence of MaSp2 (NCBI accession number: AAT75313, GI: 50363147) procured from NCBI database. In addition, as the polypeptide including two or more units of the amino-acid sequence represented by Formula 1: REP1-REP2 (1), a polypeptide which is made of the amino-acid sequence represented by SEQ ID No. 9 in which one or a plurality of amino acids is substituted, deleted, inserted, and/or added, and has a repetitive region made up of crystalline regions and amorphous regions may also be used.

Examples of the polypeptides derived from the flagelliform silk protein include polypeptides including 10 or more, preferably including 20 or more, and more preferably including 30 or more units of an amino-acid sequence represented by Formula 2: REP3 (2). In a case in which the production of recombinant protein is carried out using a microbe such as Escherichia coli as a host, the molecular weight of the polypeptide derived from the flagelliform silk protein is preferably 500 kDa or less, more preferably 300 kDa or less, and still more preferably 200 kDa or less from the viewpoint of productivity.

In Formula (2), REP3 refers to an amino-acid sequence constituted of Gly-Pro-Gly-Gly-X, and X refers to one amino acid selected from the group consisting of Ala, Ser, Tyr, and Val.

In spider silk, flagelliform silk has a significant characteristic of having a repetitive region which does not have any crystalline regions and is made of an amorphous region. Major dragline silk and the like have a repetitive region made up of crystalline regions and amorphous regions and are thus assumed to have both a high stress and a high stretching property. On the other hand, flagelliform silk has a stress that is poorer than that of major dragline silk, but has a favorable stretching property. This is considered to be due to the fact that a majority of flagelliform silk is constituted of an amorphous region.

Examples of the polypeptide including 10 or more units of the amino-acid sequence represented by Formula 2: REP3 (2) include flagelliform silk protein-derived recombinant protein having an amino-acid sequence represented by SEQ ID No. 10. The amino-acid sequence represented by SEQ ID No. 10 is an amino-acid sequence obtained by combining an amino-acid sequence of the 1220^{th} residue through the 1659^{th} residue from an N terminal that corresponds to a repeat portion and a motif of a partial sequence of a flagelliform silk protein of Nephilaclavipes (NCBI accession number: AAF36090, GI: 7106224) procured from NCBI database (referred to as the PR1 sequence) and a C terminal amino-acid sequence of the 816^{th} residue through the 907^{th} residue from a C terminal of a partial sequence of a flagelliform silk protein of Nephilaclavipes (NCBI accession number: AAC38847, GI: 2833649) procured from NCBI database and adding the amino-acid sequence (SEQ ID No. 4) made up of an initiation codon, a His10 tag, and an HRV3C protease recognition site to an N terminal of the combined sequence. In addition, as the polypeptide including 10 or more units of the amino-acid sequence represented by Formula 2: REP3 (2), a polypeptide which is made of the amino-acid sequence represented by SEQ ID No. 10 in which one or a plurality of amino acids is substituted, deleted, inserted, and/or added, and has a repetitive region made of an amorphous region may also be used.

The polypeptide can be produced using a host that is transformed by an expression vector containing a gene that codes the polypeptide. A method for producing the gene is not particularly limited, and the gene is produced by amplifying a gene that codes natural spider silk protein from a spider-derived cell by means of a polymerase chain reaction (PCR) or the like and cloning the gene or is chemically synthesized. A method for chemically synthesizing the gene is not particularly limited, and, for example, the gene can be synthesized by linking oligonucleotides that are automatically synthesized with AKTAoligopilot plus 10/100 (manufactured by GE Healthcare Company Japan) or the like by means of PCR on the basis of the amino-acid sequence information of natural spider silk protein procured from the web database of NCBI or the like. At this time, in order to facilitate the refining or confirmation of protein, a gene that codes a protein made of an amino-acid sequence obtained by adding an amino-acid sequence made up of an initiation codon and a His 10 tag to an N terminal of the amino-acid sequence may be synthesized.

As the expression vector, it is possible to use a plasmid, a phage, a virus, or the like which is capable of expressing proteins from DNA sequences. The plasmid-type expression vector is not particularly limited as long as the expression vector is capable of expressing the target gene in host cells and is self-amplifiable. For example, in a case in which Escherichia coli Rosetta (DE3) is used as a host, it is possible to use a pET22b(+) plasmid vector, a pCold plasmid vector, or the like. Among these, from the viewpoint of the productivity of protein, the pET22b(+) plasmid vector is preferably used. As the host, it is possible to use, for example, an animal cell, a plant cell, a microbe, or the like.

The polypeptide that is used in the present invention is preferably a polypeptide derived from ADF3 which is one of the two important dragline silk proteins of Araneus diadematus. This polypeptide, basically, has a high strength-elongation degree and a high toughness and also has an advantage of ease of synthesis.

### Examples

Hereinafter, the present invention will be described in more detail using examples, but the present invention is not limited to these examples within the scope of the technical concept of the present invention.

### <Gene Synthesis>

### (1) Synthesis of Gene ofADF3Kai

A partial amino-acid sequence of ADF3 which is one of the two important dragline silk proteins of Araneus diadematus (NCBI accession number: AAC47010, GI: 1263287) was acquired from the web database of NCBI, and the synthesis of a gene that codes an amino-acid sequence (SEQ ID No. 5) obtained by adding (SEQ ID No. 4) made up of an initiation codon, a His 10 tag, and a Human rhinovirus 3C protease (HRV3C protease) recognition site to an N terminal of the same sequence was entrusted to GenScript Japan Inc. As a result, a pUC57 vector (having an NdeI site at right upstream region of a 5' terminal of the gene and an Xba I site at right downsteam region of the 5' terminal) into which a gene of ADF3Kai made of a base sequence represented by SEQ ID No. 6 was introduced was acquired. Then, a restriction enzyme treatment was carried out on the same gene using Nde I and EcoR I, and the gene was recombined to a pET22b(+) expression vector.

### (2) Synthesis of Gene of ADF3Kai-noNR

The above-obtained pET22b(+) expression vector into which the gene of ADF3Kai was introduced was used as a template, and a codon GTG corresponding to the 543^{rd} amino acid residue valine (Val) in the amino-acid sequence (SEQ ID No. 5) of ADF3Kai was mutated to a termination codon TAA by means of site-directed mutagenesis using PrimeStar MutagenesisBasal Kit (manufactured by Takara Bio Inc.), thereby constructing a gene of ADF3Kai-noNR represented by SEQ ID No. 11 on pET22b(+). The accuracy of the mutagenesis was confirmed from a sequence reaction for which 3130xlGenetic Analyzer (Applied Biosystems) was used. Meanwhile, the amino-acid sequence of ADF3Kai-noNR is as indicated by SEQ ID No. 7.

### <Expression of Protein>

The pET22b(+) expression vector including a gene sequence of the above-obtained ADF3Kai-noNR was transformed to Escherichia coli Rosetta (DE3). The obtained single colony was cultured for 15 hours in a 2 mL LB culture medium including ampicillin, 1.4 mL of the resulting culture liquid was added to a 140 mL LB culture medium including ampicillin, and the culture liquid was cultured under conditions of 37°C and 200 rpm until OD₆₀₀ of the culture liquid reached 3.5. Next, the culture liquid having OD₆₀₀ of 3.5 was added to a 7 L 2xYT culture medium including ampicillin together with 140 mL of 50% glucose and was further cultured until OD₆₀₀ reached 4.0. Then, isopropyl-β-thiogalactopyranoside (IPTG) was added to the obtained culture liquid having OD₆₀₀ of 4.0 so that the final concentration reached 0.5 mM, thereby inducing protein expression. After two hours elapsed from the addition of IPTG, the culture liquid was centrifugally separated, and a bacterial body was collected. As a result of electrophoresing protein solutions prepared from the culture liquid before that addition of IPTG and after the addition of IPTG in polyacrylamide gels, bands having a target size were observed depending on the addition of IPTG, and it was confirmed that target protein was expressed. Escherichia coli that expressed the protein of ADF3Kai-noNR was stored in a freezer (-20°C).

### <Polypeptide Preparation Example>

(I) Approximately 4.5 g of the bacterial body of Escherichia coli that expressed the protein of ADF3Kai-noNR and 30 mL of a buffer solution AI (20mM of Tris-HCl, pH 7.4) were added to a centrifuge tube (50 mL), the bacterial body was dispersed using a mixer (manufactured by General Electric, SI-0286, level 10), then, centrifugal separation (10,000 rpm, 10 minutes, room temperature) was carried out using a centrifugal separator (manufactured by Tomy Seiko Co., Ltd., MX-305), and the supernatant was removed.
(II) 30 mL of a buffer solution AI and 0.3 mL of 0.1 M PMSF (dissolved using isopropanol) were added to the precipitate (bacterial body) obtained by the centrifugal separation and were dispersed for three minutes using the mixer (level 10) manufactured by General Electric. After that, the bacterial body was crushed using an ultrasonic crusher (manufactured by Sonic & Materials, Inc., VCX500), and centrifugal separation (10,000 rpm, 10 minutes, room temperature) was carried out.
(III) 30 mL of the buffer solution AI was added to the precipitate obtained by the centrifugal separation, was dispersed for three minutes using a mixer (manufactured by IKA, T18 basic ULTRA-TURRAX, level 2) manufactured by General Electric, then, centrifugal separation (10,000 rpm, 10 minutes, room temperature) was carried out using the centrifugal separator manufactured by Tomy Seiko Co., Ltd., and the supernatant was removed.
(IV) A 7.5 M urea buffer solution I (7.5 M of urea, 10 mM of sodium dihydrogen phosphate, 20 mM of NaCl, 1 mM of Tris-HCl, pH 7.0) was added to the centrifuge tube from which the supernatant was removed, and the precipitation was favorably dispersed using the ultrasonic crusher manufactured by SMT (level 7). After that, the precipitate was dissolved for 120 minutes using a shaker manufactured by Taitec Corporation (200 rpm, 60°C). A protein solution after the dissolution was centrifugally separated (11,000xg, 10 minutes, room temperature) using the centrifugal separator manufactured by Tomy Seiko Co., Ltd., and the supernatant was dialyzed using a dialysis tube (manufactured by Sanko Junyaku Co., Ltd., cellulose tube 36/32). White agglomerated protein obtained after the dialysis was collected by means of centrifugal separation, water was removed using a freeze dryer, and freeze-dried powder was collected. The degree of refining of the target protein ADF3Kai-noNR in the obtained freeze-dried powder was confirmed by image-analyzing the results of the polyacrylamide gel electrophoresis (CBB dyeing) of the powder using Totallab (Nonlinear Dynamics Ltd.). As a result, the degree of refining of ADF3Kai-noNR was approximately 85%.

### (Example 1)

### <Method for Producing Molded Article>

1.35 g of the freeze-dried powder obtained in the "polypeptide preparation example" (hereinafter, referred to as "sample") was weighed, and this sample was introduced into the through hole of the die 2 (a cylindrical die having a rectangular through hole having a 35 mm×15 mm cross section) of the press molding machine 10 illustrated in Fig. 1. At this time, the sample was added so that it became uniform thickness. After the entire sample was introduced, the heating of the die 2 was initiated, and the upper side pin 4 and the lower side pin 6 were inserted into the through hole using a hand presser (manufactured by NPa System Co., Ltd., NT-100H-V09), thereby pressing the sample. At this time, the pressurization condition of the sample was controlled to be 40 kN. The heating was stopped when the temperature of the sample reached 200°C, the sample was cooled using a spot cooler (manufactured by Trusco Nakayama Corporation, TS-25EP-1) and was removed when the temperature of the sample reached 50°C, and deburring was carried out, thereby obtaining a 35 mm× 15 mm×2 mm rectangular molded article.

### <Methods for Measuring Bending Elastic Modulus and Bending Strength>

After the obtained molded article was left to stand for one day in a constant temperature and humidity tank (manufactured by ESPEC Corp., LHL-113) under conditions of 20°C/65%RH, the following measurements were carried out.

That is, a three-point bending test was carried out using a basket jig in an autograph (manufactured by Shimadzu Corporation, AG-X plus). A load cell used was 50 kN. At this time, the distance between the support points for three-point bending was fixed to 27 mm, and the measurement rate was set to 1 mm/minute. In addition, the size of the molded article was measured using a microcaliper, and the molded article was installed in the jig and was measured. The bending elastic modulus was obtained from a displacement (strain) of up to 0.05 to 0.25%.

### <Method for Measuring Transparency 1 >

For the obtained molded articles, the transparency in the thickness direction (2 mm-thick) was visually measured, and molded articles that were highly transparent as a whole were evaluated as A, molded articles that were transparent, but included a small number of semitransparent or opaque portions were evaluated as B, and molded articles that were not transparent were evaluated as C. That is, the transparency was confirmed from whether or not the logo was visible when the molded article was placed on paper on which the logo of Spiber Inc. was printed.

### (Comparative Examples 1 and 2)

35 mm×15 mm×2 mm rectangular formed articles were obtained in the same manner as in Example 1 using polycarbonate (PC) and poly(methyl methacrylate) (PMMA). These formed articles were left to stand for one day in the constant temperature and humidity tank (manufactured by ESPEC Corp., LHL-113) under conditions of 20°C/65%RH, and then the bending elastic moduli, the bending strengths, and the transparency were measured in the same manner as in Example 1.

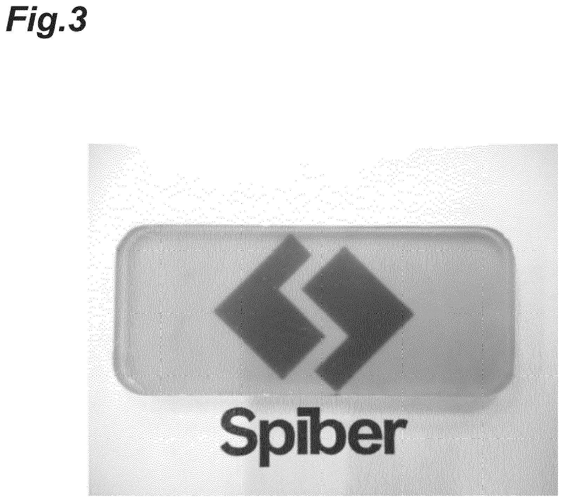

The results of the example and the comparative examples are summarized in Table 1. A photograph of the molded article used to evaluate the transparency of Example 1 is illustrated in Fig. 3.

**[Table 1]**

| | Bending Elastic Modulus (GPa) | Bending Strength (MPa) | Transparency |
|---|---|---|---|
| Example 1 | 5.39 | 74 | A |
| Comparative Example 1 (PC) | 2.68 | 97.3 | A |
| Comparative Example 2 (PMMA) | 3.2 | 105 | A |

### (Examples 2 to 18)

In Example 1, the heating was stopped when the temperature of the sample reached 200°C, the sample was cooled using the spot cooler and was taken out when the temperature of the sample reached 50°C. That is, the heating temperature (X) was 200°C, and the annealing time (Y) was 0 minutes since the rapid cooling was initiated immediately after the heating temperature was reached. Molded articles were obtained in the same manner as in Example 1 except for the fact that the heating temperature (X) and the annealing time (Y) were changed as shown in Table 2 and the pressurization condition was set to 30 kN. For the obtained molded articles, their transparencies were evaluated in the same manner as in Example 1 and were summarized in Table 2.

**[Table 2]**

| | Heating Temperature X (°C) | Annealing Time Y (Minutes) | Transparency |
|---|---|---|---|
| Example 2 | 110 | 0 | B |
| Example 3 | 120 | 0 | B |
| Example 4 | 130 | 0 | A |
| Example 5 | 140 | 0 | A |
| Example 6 | 160 | 0 | A |
| Example 7 | 180 | 0 | A |
| Example 8 | 90 | 5 | B |
| Example 9 | 100 | 5 | A |
| Example 10 | 110 | 5 | A |
| Example 11 | 120 | 5 | A |
| Example 12 | 90 | 15 | B |
| Example 13 | 120 | 15 | A |
| Example 14 | 80 | 30 | B |
| Example 15 | 90 | 30 | A |
| Example 16 | 100 | 30 | A |
| Example 17 | 110 | 30 | A |
| Example 18 | 120 | 30 | A |

### (Examples 19 to 24)

Molded articles were obtained in the same manner as in Example 1 except for the fact that the heating temperature (X), the annealing time (Y), and the pressurization condition were set as shown in Table 3. For the obtained molded articles, their transparencies were evaluated in the same manner as in Example 1 and were summarized in Table 3.

**[Table 3]**

| | Heating Temperature X(°C) | Annealing Time Y (Minutes) | Pressing Condition (kN) | Transparency |
|---|---|---|---|---|
| Example 19 | 90 | 0 | 40 | B |
| Example 20 | 100 | 0 | 40 | A |
| Example 21 | 110 | 0 | 40 | A |
| Example 22 | 120 | 0 | 40 | A |
| Example 23 | 90 | 0 | 50 | B |
| Example 24 | 100 | 0 | 50 | A |

For Examples 16 to 18 and 5 to 7, the bending elastic moduli and the bending strengths were measured in the same manner as in Example 1, and the transmittance was measured on the basis of the following method for measuring transparency 2. The results are shown in Table 4.

### <Method for Measuring Transparency 2>

The transmittance was obtained using an UV-Vis spectrophotometer (manufactured by Shimadzu Corporation, UV-2600) in a wavenumber range of 220 to 800 nm for a cumulative time of 0.1 seconds.

**[Table 4]**

| | Heating Temperature X(°C) | Annealing Time Y (Minutes) | Bending Elastic Modulus (GPa) | Bending. Strength (MPa) | Transmittance (%) |
|---|---|---|---|---|---|
| Example 16 | 100 | 30 | 6.6 | 64.6 | 54.1 |
| Example 17 | 110 | 30 | 6.3 | 67.1 | 54.5 |
| Example 18 | 120 | 30 | 5.9 | 62.5 | 56.6 |
| Example 5 | 140 | 0 | 5.2 | 52.1 | 53.8 |
| Example 6 | 160 | 0 | 5.8 | 56.7 | 54.3 |
| Example 7 | 180 | 0 | 5.7 | 63.5 | 55.2 |

### Reference Signs List

2: DIE, 4: UPPER SIDE PIN, 6: LOWER SIDE PIN, 8a: COMPOSITION BEFORE HOT PRESSING, 8b: COMPOSITION AFTER HOT PRESSING, 10: PRESS MOLDING MACHINE

## Claims

1. A molded article of a composition comprising a polypeptide, wherein the molded article is **characterised by** having more than 4.3 GPa of a bending elastic modulus and transparency.

2. The molded article according to Claim 1, wherein the polypeptide comprises at least one kind selected from the group consisting of natural spider silk protein and a polypeptide derived from natural spider silk protein.

3. The molded article of a composition comprising a polypeptide according to Claims 1 or 2,
wherein the polypeptide includes two or more units of an amino acid sequence represented by Formula 1: REP1-REP2, and
wherein
the amino acid sequences are identical or different from each other,
REP1 represents polyalanine, and
REP2 represents an amino acid sequence made of 10 to 200 residues of amino acid,
wherein the total number of glycine, serine, glutamine, and alanine in the amino acid sequence is 40% or more.

4. The molded article according to Claims 1 to 3, wherein the molded article is a hot press-molded article.

5. A method for producing the molded article according to any one of Claims 1 to 4, the method comprising a step of hot pressing the composition comprising the polypeptide.

## Patentansprüche

1. Formartikel aus einer Zusammensetzung, die ein Polypeptid umfasst, wobei der Formartikel **dadurch gekennzeichnet ist, dass** er ein Biegeelastizitätsmodul von mehr als 4,3 GPa und Transparenz aufweist.

2. Formartikel gemäß Anspruch 1, wobei das Polypeptid mindestens eine Art umfasst, die aus der Gruppe ausgewählt ist, bestehend aus natürlichem Spinnenseidenprotein und einem Polypeptid, das von natürlichem Spinnenseidenprotein stammt.

3. Formartikel aus einer Zusammensetzung, die ein Polypeptid umfasst, gemäß Anspruch 1 oder 2,
wobei das Polypeptid zwei oder mehrere Einheiten einer Aminosäuresequenz, die durch die Formel 1 : REP1-REP2 dargestellt ist, enthält, und
wobei die Aminosäuresequenzen gleich oder voneinander verschieden sind,
REP1 Polyalanin darstellt und
REP2 eine Aminosäuresequenz aus 10 bis 200 Aminosäureresten darstellt,
wobei die Gesamtanzahl von Glycin, Serin, Glutamin und Alanin in der Aminosäuresequenz 40% oder mehr beträgt.

4. Formartikel gemäß mindestens einem der Ansprüche 1 bis 3, wobei der Formartikel ein heißgepresster Artikel ist.

5. Verfahren zur Herstellung des Formartikels gemäß mindestens einem der Ansprüche 1 bis 4, wobei das Verfahren einen Schritt des Heißpressens der Zusammensetzung, die das Polypeptid umfasst, umfasst.

## Revendications

1. Article moulé d'une composition comprenant un polypeptide, dans lequel l'article moulé est **caractérisé en ce qu'**il présente plus de 4,3 GPa d'un module élastique en flexion et une transparence.

2. Article moulé selon la revendication 1, dans lequel le polypeptide comprend au moins un type sélectionné dans le groupe consistant en une protéine de soie d'araignée naturelle et un polypeptide dérivé d'une protéine de soie d'araignée naturelle.

3. Article moulé d'une composition comprenant un polypeptide selon les revendications 1 ou 2,
dans lequel le polypeptide inclut deux ou plus de deux motifs d'une séquence d'acides aminés représentée par la formule 1 : REP1-REP2, et
dans lequel
les séquences d'acides aminés sont identiques les unes aux autres ou différentes les unes des autres,
REP1 représente une polyalanine, et
REP2 représente une séquence d'acides aminés faite de 10 à 200 résidus d'acide aminé,
dans lequel le nombre total de glycine, de sérine, de glutamine, et d'alanine dans la séquence d'acides aminés est de 40 % ou plus.

4. Article moulé selon les revendications 1 à 3, dans lequel l'article moulé est un article moulé par pressage à chaud.

5. Procédé de production de l'article moulé selon l'une quelconque des revendications 1 à 4, le procédé comprenant une étape de pressage à chaud de la composition comprenant le polypeptide.


### Sequence Listing

### SEQUENCE LISTING

<110> Spiber Inc.
   KOJIMA INDUSTRIES CORPORATION
   TEKUNOHAMA CO., LTD
   MURORAN INSTITUTE OF TECHNOLOGY
<120> MOLDED MATERIAL
<130> 2015PS004W01
<160> 11
<170> PatentIn version 3.5
<210> 1
   <211> 50
   <212> PRT
   <213> Araneus diadematus
<400> 1
   Ser Gly Cys Asp Val Leu Val Gln Ala Leu Leu Glu Val Val Ser Ala
   1 5 10 15
   Leu Val Ser Ile Leu Gly Ser Ser Ser Ile Gly Gln Ile Asn Tyr Gly
   20 25 30
   Ala Ser Ala Gln Tyr Thr Gln Met Val Gly Gln Ser Val Ala Gln Ala
   35 40 45
   Leu Ala
   50
<210> 2
   <211> 30
   <212> PRT
   <213> Araneus diadematus
<400> 2
   Ser Gly Cys Asp Val Leu Val Gln Ala Leu Leu Glu Val Val Ser Ala
   1 5 10 15
   Leu Val Ser Ile Leu Gly Ser Ser Ser Ile Gly Gln Ile Asn
   20 25 30
<210> 3
   <211> 21
   <212> PRT
   <213> Araneus diadematus
<400> 3
   Ser Gly Cys Asp Val Leu Val Gln Ala Leu Leu Glu Val Val Ser Ala
   1 5 10 15
   Leu Val Ser Ile Leu
   20
<210> 4
   <211> 24
   <212> PRT
   <213> Artificial Sequence
<220>
   <223> His tag and start codon
<400> 4
   Met His His His His His His His His His His Ser Ser Gly Ser Ser
   1 5 10 15
   Leu Glu Val Leu Phe Gln Gly Pro
   20
<210> 5
   <211> 660
   <212> PRT
   <213> Artificial Sequence
<220>
   <223> recombinant spider silk protein ADF3Kai
<400> 5
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gly Gln Gly Pro Tyr Gly Pro Gly Ser Ser Ala Ala Ala Ala Ala
           115                 120                 125
   Ala Gly Gly Asn Gly Pro Gly Ser Gly Gln Gln Gly Ala Gly Gln Gln
       130                 135                 140
   Gly Pro Gly Gln Gln Gly Pro Gly Ala Ser Ala Ala Ala Ala Ala Ala
   145                 150                 155                 160
   Gly Gly Tyr Gly Pro Gly Ser Gly Gln Gln Gly Pro Gly Gln Gln Gly
                   165                 170                 175
   Pro Gly Gly Gln Gly Pro Tyr Gly Pro Gly Ala Ser Ala Ala Ala Ala
               180                 185                 190
   Ala Ala Gly Gly Tyr Gly Pro Gly Ser Gly Gln Gly Pro Gly Gln Gln
           195                 200                 205
   Gly Pro Gly Gly Gln Gly Pro Tyr Gly Pro Gly Ala Ser Ala Ala Ala
       210                 215                 220
   Ala Ala Ala Gly Gly Tyr Gly Pro Gly Ser Gly Gln Gln Gly Pro Gly
   225                 230                 235                 240
   Gln Gln Gly Pro Gly Gln Gln Gly Pro Gly Gly Gln Gly Pro Tyr Gly
                   245                 250                 255
   Pro Gly Ala Ser Ala Ala Ala Ala Ala Ala Gly Gly Tyr Gly Pro Gly
               260                 265                 270
   Tyr Gly Gln Gln Gly Pro Gly Gln Gln Gly Pro Gly Gly Gln Gly Pro
           275                 280                 285
   Tyr Gly Pro Gly Ala Ser Ala Ala Ser Ala Ala Ser Gly Gly Tyr Gly
       290                 295                 300
   Pro Gly Ser Gly Gln Gln Gly Pro Gly Gln Gln Gly Pro Gly Gly Gln
   305                 310                 315                 320
   Gly Pro Tyr Gly Pro Gly Ala Ser Ala Ala Ala Ala Ala Ala Gly Gly
                   325                 330                 335
   Tyr Gly Pro Gly Ser Gly Gln Gln Gly Pro Gly Gln Gln Gly Pro Gly
               340                 345                 350
   Gln Gln Gly Pro Gly Gln Gln Gly Pro Gly Gly Gln Gly Pro Tyr Gly
           355                 360                 365
   Pro Gly Ala Ser Ala Ala Ala Ala Ala Ala Gly Gly Tyr Gly Pro Gly
       370                 375                 380
   Ser Gly Gln Gln Gly Pro Gly Gln Gln Gly Pro Gly Gln Gln Gly Pro
   385                 390                 395                 400
   Gly Gln Gln Gly Pro Gly Gln Gln Gly Pro Gly Gln Gln Gly Pro Gly
                   405                 410                 415
   Gln Gln Gly Pro Gly Gln Gln Gly Pro Gly Gln Gln Gly Pro Gly Gly
               420                 425                 430
   Gln Gly Ala Tyr Gly Pro Gly Ala Ser Ala Ala Ala Gly Ala Ala Gly
           435                 440                 445
   Gly Tyr Gly Pro Gly Ser Gly Gln Gln Gly Pro Gly Gln Gln Gly Pro
       450                 455                 460
   Gly Gln Gln Gly Pro Gly Gln Gln Gly Pro Gly Gln Gln Gly Pro Gly
   465                 470                 475                 480
   Gln Gln Gly Pro Gly Gln Gln Gly Pro Gly Gln Gln Gly Pro Tyr Gly
                   485                 490                 495
   Pro Gly Ala Ser Ala Ala Ala Ala Ala Ala Gly Gly Tyr Gly Pro Gly
               500                 505                 510
   Ser Gly Gln Gln Gly Pro Gly Gln Gln Gly Pro Gly Gln Gln Gly Pro
           515                 520                 525
   Gly Gly Gln Gly Pro Tyr Gly Pro Gly Ala Ala Ser Ala Ala Val Ser
       530                 535                 540
   Val Gly Gly Tyr Gly Pro Gln Ser Ser Ser Val Pro Val Ala Ser Ala
   545                 550                 555                 560
   Val Ala Ser Arg Leu Ser Ser Pro Ala Ala Ser Ser Arg Val Ser Ser
                   565                 570                 575
   Ala Val Ser Ser Leu Val Ser Ser Gly Pro Thr Lys His Ala Ala Leu
               580                 585                 590
   Ser Asn Thr Ile Ser Ser Val Val Ser Gln Val Ser Ala Ser Asn Pro
           595                 600                 605
   Gly Leu Ser Gly Cys Asp Val Leu Val Gln Ala Leu Leu Glu Val Val
       610                 615                 620
   Ser Ala Leu Val Ser Ile Leu Gly Ser Ser Ser Ile Gly Gln Ile Asn
   625                 630                 635                 640
   Tyr Gly Ala Ser Ala Gln Tyr Thr Gln Met Val Gly Gln Ser Val Ala
                   645                 650                 655
   Gln Ala Leu Ala
               660
<210> 6
   <211> 1983
   <212> DNA
   <213> Artificial Sequence
<220>
   <223> recombinant DNA of spider silk protein gene ADF3Kai
<400> 6
   atgcatcacc atcatcatca tcaccaccac cattcctcgg gctcatcctt ggaagtgtta      60
   tttcaaggac cagcacgagc cggttcggga caacaagggc ctggccagca gggcccaggt     120
   caacaagggc caggacagca gggtccttat gggcccggcg caagcgcagc agctgcggcc     180
   gctggtggct atggtcctgg ctccggtcaa cagggccctt cgcaacaagg tcccgggcag     240
   caaggtcctg gtggccaggg tccctacggg ccgggggcga gtgcggcagc agccgctgca     300
   ggcggttatg gtccaggaag cggacagcaa ggtccgggag gtcaaggtcc gtatggccca     360
   ggctctagcg cggctgccgc tgccgcgggt ggcaacggac cagggagcgg acaacagggc     420
   gcgggacaac agggtccagg acagcaaggc ccaggggcgt cggcggctgc agcggcggcc     480
   ggaggctatg gacccggctc aggacaacag ggaccgggtc aacaaggacc cggtggccaa     540
   ggcccctatg gcccgggcgc cagcgcggcc gcagccgccg cgggcgggta cggccccggt     600
   agcggccagg gaccaggtca gcaggggcca ggaggtcagg gcccatacgg tccgggcgca     660
   tccgcggcgg cggcagcggc aggtggctac ggtcccggaa gcggccaaca ggggccaggg     720
   caacaaggac caggacaaca aggtcctggg ggccaaggac cgtatggacc aggagcatca     780
   gctgcagccg cggcagctgg cggttacggt ccaggctacg gccagcaggg tccgggtcag     840
   cagggaccgg gaggccaggg gccttatggc cctggcgctt ccgcagccag tgccgcttct     900
   ggaggatacg ggccgggaag cggtcagcaa ggccctggcc aacaaggacc tggaggccaa     960
   gggccctacg gcccaggagc ctcggcagcc gcagctgccg caggtgggta tgggccaggt    1020
   agcgggcaac aagggccggg tcagcaagga ccggggcaac agggacctgg gcagcaagga    1080
   cccgggggtc aaggcccgta cggacctggt gcgtctgcag ctgctgctgc ggctggtgga    1140
   tatggtccgg gatcggggca gcagggtccc ggtcagcagg gccctggtca gcaagggcca    1200
   ggccaacagg gacccggaca acaaggcccg ggtcaacagg gtcctggaca gcaggggccg   1260
   ggccaacaag gccctgggca acagggtccg gggggacagg gggcctatgg gcctggcgca   1320
   tctgccgccg ctggcgcagc cggtgggtac gggcctgggt caggtcaaca ggggcctggt   1380
   caacaaggcc ccgggcaaca gggccccggc cagcaaggtc cagggcagca gggcccggga   1440
   cagcaagggc ctggacaaca ggggcccgga cagcagggac cttacgggcc cggtgcgagc   1500
   gcagcggccg ccgccgcagg gggatatggc cccggatcgg gccagcaggg accaggccag   1560
   caaggacctg gccaacaggg cccggggggt caggggccgt atggtcccgg cgctgcaagt   1620
   gctgcagtgt ccgttggagg ttacggccct cagtcttcgt ctgttccggt ggcgtccgca   1680
   gttgcgagta gactgtcttc acctgctgct tcatcgcgag tatcgagcgc tgtttcgtct   1740
   cttgtctcgt cgggtcccac gaaacatgcc gccctttcaa atacgatttc atctgtagtg   1800
   tcccaagtta gtgcaagtaa cccggggtta tccggatgcg acgttctcgt tcaggcactc   1860
   ctagaagtag tatccgcgtt ggtgagcatc ttaggcagct cctcgatagg tcaaataaac   1920
   tatggtgctt cagcccagta tacacagatg gtgggacaga gcgtcgcgca ggcattggct   1980
   taa                                                                  1983
<210> 7
   <211> 542
   <212> PRT
   <213> Artificial Sequence
<220>
   <223> recombinant DNA of spider silk protein gene ADF3Kai
<400> 7
   Met His His His His His His His His His His Ser Ser Gly Ser Ser
   1               5                   10                  15
   Leu Glu Val Leu Phe Gln Gly Pro Ala Arg Ala Gly Ser Gly Gln Gln
               20                  25                  30
   Gly Pro Gly Gln Gln Gly Pro Gly Gln Gln Gly Pro Gly Gln Gln Gly
           35                  40                  45
   Pro Tyr Gly Pro Gly Ala Ser Ala Ala Ala Ala Ala Ala Gly Gly Tyr
       50                  55                  60
   Gly Pro Gly Ser Gly Gln Gln Gly Pro Ser Gln Gln Gly Pro Gly Gln
   65                  70                  75                  80
   Gln Gly Pro Gly Gly Gln Gly Pro Tyr Gly Pro Gly Ala Ser Ala Ala
                   85                  90                  95
   Ala Ala Ala Ala Gly Gly Tyr Gly Pro Gly Ser Gly Gln Gln Gly Pro
               100                 105                 110
   Gly Gly Gln Gly Pro Tyr Gly Pro Gly Ser Ser Ala Ala Ala Ala Ala
           115                 120                 125
   Ala Gly Gly Asn Gly Pro Gly Ser Gly Gln Gln Gly Ala Gly Gln Gln
       130                 135                 140
   Gly Pro Gly Gln Gln Gly Pro Gly Ala Ser Ala Ala Ala Ala Ala Ala
   145                 150                 155                 160
   Gly Gly Tyr Gly Pro Gly Ser Gly Gln Gln Gly Pro Gly Gln Gln Gly
                   165                 170                 175
   Pro Gly Gly Gln Gly Pro Tyr Gly Pro Gly Ala Ser Ala Ala Ala Ala
               180                 185                 190
   Ala Ala Gly Gly Tyr Gly Pro Gly Ser Gly Gln Gly Pro Gly Gln Gln
           195                 200                 205
   Gly Pro Gly Gly Gln Gly Pro Tyr Gly Pro Gly Ala Ser Ala Ala Ala
       210                 215                 220
   Ala Ala Ala Gly Gly Tyr Gly Pro Gly Ser Gly Gln Gln Gly Pro Gly
   225                 230                 235                 240
   Gln Gln Gly Pro Gly Gln Gln Gly Pro Gly Gly Gln Gly Pro Tyr Gly
                   245                 250                 255
   Pro Gly Ala Ser Ala Ala Ala Ala Ala Ala Gly Gly Tyr Gly Pro Gly
               260                 265                 270
   Tyr Gly Gln Gln Gly Pro Gly Gln Gln Gly Pro Gly Gly Gln Gly Pro
           275                 280                 285
   Tyr Gly Pro Gly Ala Ser Ala Ala Ser Ala Ala Ser Gly Gly Tyr Gly
       290                 295                 300
   Pro Gly Ser Gly Gln Gln Gly Pro Gly Gln Gln Gly Pro Gly Gly Gln
   305                 310                 315                 320
   Gly Pro Tyr Gly Pro Gly Ala Ser Ala Ala Ala Ala Ala Ala Gly Gly
                   325                 330                 335
   Tyr Gly Pro Gly Ser Gly Gln Gln Gly Pro Gly Gln Gln Gly Pro Gly
               340                 345                 350
   Gln Gln Gly Pro Gly Gln Gln Gly Pro Gly Gly Gln Gly Pro Tyr Gly
           355                 360                 365
   Pro Gly Ala Ser Ala Ala Ala Ala Ala Ala Gly Gly Tyr Gly Pro Gly
       370                 375                 380
   Ser Gly Gln Gln Gly Pro Gly Gln Gln Gly Pro Gly Gln Gln Gly Pro
   385                 390                 395                 400
   Gly Gln Gln Gly Pro Gly Gln Gln Gly Pro Gly Gln Gln Gly Pro Gly
                   405                 410                 415
   Gln Gln Gly Pro Gly Gln Gln Gly Pro Gly Gln Gln Gly Pro Gly Gly
               420                 425                 430
   Gln Gly Ala Tyr Gly Pro Gly Ala Ser Ala Ala Ala Gly Ala Ala Gly
           435                 440                 445
   Gly Tyr Gly Pro Gly Ser Gly Gln Gln Gly Pro Gly Gln Gln Gly Pro
       450                 455                 460
   Gly Gln Gln Gly Pro Gly Gln Gln Gly Pro Gly Gln Gln Gly Pro Gly
   465                 470                 475                 480
   Gln Gln Gly Pro Gly Gln Gln Gly Pro Gly Gln Gln Gly Pro Tyr Gly
                   485                 490                 495
   Pro Gly Ala Ser Ala Ala Ala Ala Ala Ala Gly Gly Tyr Gly Pro Gly
               500                 505                 510
   Ser Gly Gln Gln Gly Pro Gly Gln Gln Gly Pro Gly Gln Gln Gly Pro
           515                 520                 525
   Gly Gly Gln Gly Pro Tyr Gly Pro Gly Ala Ala Ser Ala Ala
       530                 535                 540
<210> 8
   <211> 434
   <212> PRT
   <213> Artificial
<220>
   <223> Recombinang spider silk protein ADF4Kai
<400> 8
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
   260 265 270
   Gly Pro Gly Gly Ser Gly Gly Tyr Gly Pro Gly Ser Gln Gly Gly Ser
   275 280 285
   Gly Pro Gly Ala Ser Ala Ala Ala Ala Ala Ala Ala Ala Ser Gly Pro
   290 295 300
   Gly Gly Tyr Gly Pro Gly Ser Gln Gly Pro Ser Gly Pro Gly Tyr Gln
   305 310 315 320
   Gly Pro Ser Gly Pro Gly Ala Tyr Gly Pro Ser Pro Ser Ala Ser Ala
   325 330 335
   Ser Val Ala Ala Ser Val Tyr Leu Arg Leu Gln Pro Arg Leu Glu Val
   340 345 350
   Ser Ser Ala Val Ser Ser Leu Val Ser Ser Gly Pro Thr Asn Gly Ala
   355 360 365
   Ala Val Ser Gly Ala Leu Asn Ser Leu Val Ser Gln Ile Ser Ala Ser
   370 375 380
   Asn Pro Gly Leu Ser Gly Cys Asp Ala Leu Val Gln Ala Leu Leu Glu
   385 390 395 400
   Leu Val Ser Ala Leu Val Ala Ile Leu Ser Ser Ala Ser Ile Gly Gln
   405 410 415
   Val Asn Val Ser Ser Val Ser Gln Ser Thr Gln Met Ile Ser Gln Ala
   420 425 430
   Leu Ser
<210> 9
   <211> 355
   <212> PRT
   <213> Artificial
<220>
   <223> Recombinant spider silk protein MaSp2_N
<400> 9
   Met His His His His His His Ser Ser Gly Ser Ser Leu Glu Val Leu
   1 5 10 15
   Phe Gln Gly Pro Ala Arg Ala Gly Pro Gly Gly Tyr Arg Pro Gly Gln
               20                  25                  30
   Gln Gly Pro Ser Gly Pro Gly Ser Ala Ala Ala Ala Ala Ala Ala Ala
           35                  40                  45
   Ala Gly Pro Gly Gly Tyr Gly Pro Gly Gln Gln Gly Pro Gly Gly Tyr
       50                  55                  60
   Gly Pro Gly Gln Gln Gly Pro Ser Gly Ala Gly Ser Ala Ala Ala Ala
   65                  70                  75                  80
   Ala Ala Ala Gly Pro Gly Gln Gln Gly Leu Gly Gly Tyr Gly Pro Gly
                   85                  90                  95
   Gln Gln Gly Pro Gly Gly Tyr Gly Pro Gly Gln Gln Gly Pro Gly Gly
               100                 105                 110
   Tyr Gly Pro Gly Ser Ala Ser Ala Ala Ala Ala Ala Ala Gly Pro Gly
           115                 120                 125
   Gln Gln Gly Pro Gly Gly Tyr Gly Pro Gly Gln Gln Gly Pro Ser Gly
       130                 135                 140
   Pro Gly Ser Ala Ser Ala Ala Ala Ala Ala Ala Gly Pro Gly Gly Tyr
   145                 150                 155                 160
   Gly Pro Gly Gln Gln Gly Pro Gly Gly Tyr Ala Pro Gly Gln Gln Gly
                   165                 170                 175
   Pro Ser Gly Pro Gly Ser Ala Ala Ala Ala Ala Ala Ala Ala Arg Ala
               180                 185                 190
   Gly Pro Gly Gly Tyr Gly Pro Ala Gln Gln Gly Pro Ser Gly Pro Gly
           195                 200                 205
   Ile Ala Ala Ser Ala Ala Ser Ala Gly Pro Gly Gly Tyr Gly Pro Ala
       210                 215                 220
   Gln Gln Gly Pro Ala Gly Tyr Gly Pro Gly Ser Ala Val Ala Ala Ser
   225                 230                 235                 240
   Ala Gly Ala Gly Ser Ala Gly Tyr Gly Pro Gly Ser Gln Ala Ser Ala
                   245                 250                 255
   Ala Ala Ser Arg Leu Ala Ser Pro Asp Ser Gly Ala Arg Val Ala Ser
               260                 265                 270
   Ala Val Ser Asn Leu Val Ser Ser Gly Pro Thr Ser Ser Ala Ala Leu
   275 280 285
   Ser Ser Val Ile Ser Asn Ala Val Ser Gln Ile Gly Ala Ser Asn Pro
   290 295 300
   Gly Leu Ser Gly Cys Asp Val Leu Ile Gln Ala Leu Leu Glu Ile Val
   305 310 315 320
   Ser Ala Cys Val Thr Ile Leu Ser Ser Ser Ser Ile Gly Gln Val Asn
   325 330 335
   Tyr Gly Ala Ala Ser Gln Phe Ala Gln Val Val Gly Gln Ser Val Leu
   340 345 350
   Ser Ala Phe
   355
<210> 10
   <211> 559
   <212> PRT
   <213> Artificial
<220>
   <223> Recombinant spider silk protein Flag_92_short2
<400> 10
   Met His His His His His His His His His His Ser Ser Gly Ser Ser
   1 5 10 15
   Leu Glu Val Leu Phe Gln Gly Pro Gly Ala Gly Gly Ser Gly Pro Gly
   20 25 30
   Gly Ala Gly Pro Gly Gly Val Gly Pro Gly Gly Ser Gly Pro Gly Gly
   35 40 45
   Val Gly Pro Gly Gly Ser Gly Pro Gly Gly Val Gly Pro Gly Gly Ser
   50 55 60
   Gly Pro Gly Gly Val Gly Pro Gly Gly Ala Gly Gly Pro Tyr Gly Pro
   65 70 75 80
   Gly Gly Ser Gly Pro Gly Gly Ala Gly Gly Ala Gly Gly Pro Gly Gly
   85 90 95
   Ala Tyr Gly Pro Gly Gly Ser Tyr Gly Pro Gly Gly Ser Gly Gly Pro
   100 105 110
   Gly Gly Ala Gly Gly Pro Tyr Gly Pro Gly Gly Glu Gly Pro Gly Gly
           115                 120                 125
   Ala Gly Gly Pro Tyr Gly Pro Gly Gly Ala Gly Gly Pro Tyr Gly Pro
       130                 135                 140
   Gly Gly Ala Gly Gly Pro Tyr Gly Pro Gly Gly Glu Gly Gly Pro Tyr
   145                 150                 155                 160
   Gly Pro Gly Gly Ser Tyr Gly Pro Gly Gly Ala Gly Gly Pro Tyr Gly
                   165                 170                 175
   Pro Gly Gly Pro Tyr Gly Pro Gly Gly Glu Gly Pro Gly Gly Ala Gly
               180                 185                 190
   Gly Pro Tyr Gly Pro Gly Gly Val Gly Pro Gly Gly Gly Gly Pro Gly
           195                 200                 205
   Gly Tyr Gly Pro Gly Gly Ala Gly Pro Gly Gly Tyr Gly Pro Gly Gly
       210                 215                 220
   Ser Gly Pro Gly Gly Tyr Gly Pro Gly Gly Ser Gly Pro Gly Gly Tyr
   225                 230                 235                 240
   Gly Pro Gly Gly Ser Gly Pro Gly Gly Tyr Gly Pro Gly Gly Ser Gly
                   245                 250                 255
   Pro Gly Gly Tyr Gly Pro Gly Gly Ser Gly Pro Gly Gly Ser Gly Pro
               260                 265                 270
   Gly Gly Tyr Gly Pro Gly Gly Ser Gly Pro Gly Gly Ser Gly Pro Gly
           275                 280                 285
   Gly Tyr Gly Pro Gly Gly Ser Gly Pro Gly Gly Tyr Gly Pro Gly Gly
       290                 295                 300
   Ser Gly Pro Gly Gly Ser Gly Pro Gly Gly Tyr Gly Pro Gly Gly Ser
   305                 310                 315                 320
   Gly Pro Gly Gly Ser Gly Pro Gly Gly Tyr Gly Pro Gly Gly Ser Gly
                   325                 330                 335
   Pro Gly Gly Phe Gly Pro Gly Gly Phe Gly Pro Gly Gly Ser Gly Pro
               340                 345                 350
   Gly Gly Tyr Gly Pro Gly Gly Ser Gly Pro Gly Gly Ala Gly Pro Gly
           355                 360                 365
   Gly Val Gly Pro Gly Gly Phe Gly Pro Gly Gly Ala Gly Pro Gly Gly
       370                 375                 380
   Ala Gly Pro Gly Gly Ala Gly Pro Gly Gly Ala Gly Pro Gly Gly Ala
   385                 390                 395                 400
   Gly Pro Gly Gly Ala Gly Pro Gly Gly Ala Gly Pro Gly Gly Ala Gly
                   405                 410                 415
   Pro Gly Gly Ala Gly Gly Ala Gly Gly Ala Gly Gly Ala Gly Gly Ser
               420                 425                 430
   Gly Gly Ala Gly Gly Ser Gly Gly Thr Thr Ile Ile Glu Asp Leu Asp
           435                 440                 445
   Ile Thr Ile Asp Gly Ala Asp Gly Pro Ile Thr Ile Ser Glu Glu Leu
       450                 455                 460
   Thr Ile Ser Ala Tyr Tyr Pro Ser Ser Arg Val Pro Asp Met Val Asn
   465                 470                 475                 480
   Gly Ile Met Ser Ala Met Gln Gly Ser Gly Phe Asn Tyr Gln Met Phe
                   485                 490                 495
   Gly Asn Met Leu Ser Gln Tyr Ser Ser Gly Ser Gly Thr Cys Asn Pro
               500                 505                 510
   Asn Asn Val Asn Val Leu Met Asp Ala Leu Leu Ala Ala Leu His Cys
           515                 520                 525
   Leu Ser Asn His Gly Ser Ser Ser Phe Ala Pro Ser Pro Thr Pro Ala
       530                 535                 540
   Ala Met Ser Ala Tyr Ser Asn Ser Val Gly Arg Met Phe Ala Tyr
   545                 550                 555
<210> 11
   <211> 1629
   <212> DNA
   <213> Artificial Sequence
<220>
   <223> recombinant spider silk protein gene ADF3Kai-noNR
<400> 11
   atgcatcacc atcatcatca tcaccaccac cattcctcgg gctcatcctt ggaagtgtta      60
   tttcaaggac cagcacgagc cggttcggga caacaagggc ctggccagca gggcccaggt     120
   caacaagggc caggacagca gggtccttat gggcccggcg caagcgcagc agctgcggcc     180
   gctggtggct atggtcctgg ctccggtcaa cagggccctt cgcaacaagg tcccgggcag     240
   caaggtcctg gtggccaggg tccctacggg ccgggggcga gtgcggcagc agccgctgca     300
   ggcggttatg gtccaggaag cggacagcaa ggtccgggag gtcaaggtcc gtatggccca     360
   ggctctagcg cggctgccgc tgccgcgggt ggcaacggac cagggagcgg acaacagggc     420
   gcgggacaac agggtccagg acagcaaggc ccaggggcgt cggcggctgc agcggcggcc     480
   ggaggctatg gacccggctc aggacaacag ggaccgggtc aacaaggacc cggtggccaa     540
   ggcccctatg gcccgggcgc cagcgcggcc gcagccgccg cgggcgggta cggccccggt     600
   agcggccagg gaccaggtca gcaggggcca ggaggtcagg gcccatacgg tccgggcgca     660
   tccgcggcgg cggcagcggc aggtggctac ggtcccggaa gcggccaaca ggggccaggg     720
   caacaaggac caggacaaca aggtcctggg ggccaaggac cgtatggacc aggagcatca     780
   gctgcagccg cggcagctgg cggttacggt ccaggctacg gccagcaggg tccgggtcag     840
   cagggaccgg gaggccaggg gccttatggc cctggcgctt ccgcagccag tgccgcttct     900
   ggaggatacg ggccgggaag cggtcagcaa ggccctggcc aacaaggacc tggaggccaa     960
   gggccctacg gcccaggagc ctcggcagcc gcagctgccg caggtgggta tgggccaggt    1020
   agcgggcaac aagggccggg tcagcaagga ccggggcaac agggacctgg gcagcaagga    1080
   cccgggggtc aaggcccgta cggacctggt gcgtctgcag ctgctgctgc ggctggtgga    1140
   tatggtccgg gatcggggca gcagggtccc ggtcagcagg gccctggtca gcaagggcca    1200
   ggccaacagg gacccggaca acaaggcccg ggtcaacagg gtcctggaca gcaggggccg    1260
   ggccaacaag gccctgggca acagggtccg gggggacagg gggcctatgg gcctggcgca    1320
   tctgccgccg ctggcgcagc cggtgggtac gggcctgggt caggtcaaca ggggcctggt    1380
   caacaaggcc ccgggcaaca gggccccggc cagcaaggtc cagggcagca gggcccggga    1440
   cagcaagggc ctggacaaca ggggcccgga cagcagggac cttacggccc cggtgcgagc     1500
   gcagcggccg ccgccgcagg gggatatggc cccggatcgg gccagcaggg accaggccag    1560
   caaggacctg gccaacaggg cccggggggt caggggccgt atggtcccgg cgctgcaagt     1620
   gctgcataa                                                            1629